# FASCICULE DE BREVET EUROPEEN

(11) **EP 3 551 234 B1**
(45) Date de publication et mention de la délivrance du brevet: **04.11.2020**
(21) Numéro de dépôt: 17821708.9
(22) Date de dépôt: 11.12.2017
(51) Int. Cl.: A61L 2/24, A61L 2/26, A61B 90/70, A61B 1/12

(54) **MACHINE DE TRAITEMENT D'UN APPAREIL MEDICAL**
MASCHINE ZUR BEHANDLUNG EINER MEDIZINISCHEN VORRICHTUNG
MACHINE FOR TREATING A MEDICAL APPARATUS

(30) Priorité: 09.12.2016 FR 1662203
(43) Date de publication de la demande: 16.10.2019
(73) Titulaire: Ecolab USA Inc., St. Paul, MN 55012 (US)
(72) Inventeur: AFFRE, Christian, 13190 Allauch (FR); BIRY, Jean-François, 13090 Aix en Provence (FR); RUIZ, Sébastien, 13340 Rognac (FR)
(74) Mandataire: Plasseraud IP
(86) Numéro de dépôt international: PCT/FR2017/053488
(87) Numéro de publication internationale: WO 2018/104690

(56) Documents cités:
- EP-B1- 2 925 373
- WO-A1-2016/087244
- WO-A2-2016/186502
- CN-A- 102 974 585

## Description

L'invention est relative à une machine de traitement d'un appareil médical, ainsi qu'un ensemble comprenant une machine de traitement et un mur séparant une première enceinte à atmosphère contrôlée/non contrôlée d'une seconde enceinte à atmosphère non contrôlée/contrôlée. L'invention concerne encore un procédé de maintenance d'une machine de traitement d'un ensemble selon l'invention.

Le domaine de l'invention est celui des machines de traitement d'appareil médicaux, et plus particulièrement celui des laveurs désinfecteur d'endoscope (acronyme « *LDE* »).

L'invention s'intéresse ici plus particulièrement ici au LDE que l'homme du métier désigne habituellement par LDE de type *« pass-through »* (de l'anglais « *au travers* ») en ce que ce type de machine s'intègre au travers d'un mur de séparation. Ces machines sont destinées à être intégrées dans une ouverture d'un mur séparant une enceinte à atmosphère contrôlée (par exemple l'enceinte d'un bloc opératoire dont l'atmosphère contrôlée est typiquement purifiée par filtration et mise en suppression) d'une enceinte à atmosphère non contrôlée, tel qu'une salle de service, et susceptible de contenir des contaminants (bactéries..) en suspension dans l'air.

De manière notable, ce type de LDE présente une porte de chargement, côté enceinte à atmosphère non contrôlée, permettant par exemple le chargement d'endoscopes souillés depuis ce côté, directement dans la cuve de traitement du LDE à partir d'une première ouverture de la cuve, et une autre porte, côté atmosphère contrôlée, permettant de récupérer l'endoscope désinfecté, depuis ce côté (*i.e.* le bloc opératoire), à partir d'une seconde ouverture de la cuve, diamétralement opposée à la première ouverture. Il est également envisageable de charger l'endoscope souillé depuis l'enceinte à atmosphère contrôlée, et de le décharger côté enceinte à atmosphère non contrôlée.

Cette machine se présente sous la forme d'un mobilier d'un seul tenant, comprenant notamment :
- un bâti,
- la cuve de chargement, y compris avec ses deux portes d'accès,
- le système de traitement, présentant le ou les circuits de traitement, y compris la ou les pompes, la ou les conduites et les électrovalves, éventuellement les buses, permettant le traitement des surfaces intérieures et extérieures de l'endoscope contenu dans la cuve de traitement, à partir d'un réservoir de solution désinfectante,
- l'automate programmable assurant la commande du système de traitement, en particulier du moteur et des électrovalves du système, et pour la mise en œuvre d'un cycle de désinfection.

De manière notable, le mobilier d'un LDE de type « *pass-through* » s'intègre dans l'ouverture traversant le mur de séparation, en assurant une étanchéité relative entre les deux atmosphères, respectivement contrôlée et non contrôlée.

Certaines maintenances lourdes du LDE ne peuvent être réalisées sur site, mais imposent au contraire le retrait complet du mobilier, en vue d'une révision/réparation en atelier. Dans un tel cas, le retrait du mobilier libère totalement l'ouverture du mur de séparation, la perte d'étanchéité en résultant ne permettant plus de garantir le contrôle de la qualité de l'air dans l'enceinte à atmosphère contrôlée, telle que celle d'un bloc opératoire. Ainsi, et selon les constatations de l'inventeur, pour la conception de LDE de type « *pass through* » connue de l'état de la technique, le bloc opératoire est immobilisé après retrait du LDE, et jusqu'à la remise en place du mobilier dans l'ouverture de séparation.

Un tel type de LDE « *pass-through* » est par exemple connu du document WO 2014/083524 A1.

Le but de la présente invention est de proposer une machine de traitement d'endoscope, tel qu'un LDE dont la cuve de traitement présente deux ouvertures opposées autorisant le chargement/déchargement des deux côtés d'un mur destiné à séparer une enceinte à atmosphère contrôlée, d'une enceinte à atmosphère non contrôlée, tout en autorisant toujours le contrôle de la qualité de l'air de l'atmosphère contrôlée, même en cas de maintenance lourde, imposant un retrait de cet équipement.

Un autre but de la présente invention est de proposer, au moins selon un mode de réalisation, une telle machine qui répond à cet objectif, de sécurité accrue, limitant le nombre de manipulation pour parvenir à cet objectif.

D'autres buts et avantages apparaitront au cours de la description qui va suivre qui n'est donnée qu'à titre indicatif et qui n'a pas pour but de la limiter.

L'invention concerne tout d'abord une machine de traitement d'un appareil médical, tel qu'un laveur désinfecteur d'endoscope, destiné à être intégrer sur un mur, dit mur de séparation au niveau d'une ouverture traversante du mur de séparation, le mur étant destiné à séparer une première enceinte à atmosphère contrôlée/non contrôlée d'une seconde enceinte à atmosphère non contrôlée/contrôlée, ladite machine comportant :
- une cuve de traitement présentant une première ouverture configurée de manière à permettre le chargement/déchargement d'un appareil médical à partir de la première enceinte et une seconde ouverture configurée de manière à permettre le chargement/déchargement d'un appareil médical à partir de la seconde enceinte,
- un premier système de porte assurant la fermeture de manière étanche de la première ouverture,
- un deuxième système de porte assurant la fermeture de manière étanche de la seconde ouverture,
- un système de traitement d'une appareil médical présentant un ou plusieurs circuits de traitement, y compris une ou plusieurs pompes, une ou plusieurs électrovalves, permettant le traitement des surfaces intérieures et extérieures de l'appareil médical contenu dans la cuve de traitement par immersion et/ou par aspersion, à partir d'une solution désinfectante prélevée dans un réservoir hors de la cuve de traitement,
- un système automate programmable assurant la commande du système de traitement, en particulier du moteur et des électrovalves du système, configuré pour la mise en œuvre d'un cycle de désinfection d'un appareil médical.

Selon l'invention, ladite machine comprend :
- une interface de cloisonnement destinée à être fixée à demeure au mur et configurée de manière à obturer l'ouverture du mur, ladite interface de cloisonnement comportant au moins une ouverture traversant destinée à être en regard de la deuxième ouverture de la cuve, au moins en position de fonctionnement de la machine de traitement, le deuxième système de porte étant solidaire de ladite interface de cloisonnement, assurant dans sa position de fermeture l'obturation de ladite ouverture de l'interface de cloisonnement,
- une partie mobile, amovible de l'interface de cloisonnement comportant un châssis autoportant, et embarquant au moins en partie la ou les pompes et les électrovalves dudit système de traitement de l'appareil médical ainsi que ledit automate programmable, qui en position de fonctionnement de la machine de traitement est configuré pour se positionner à proximité immédiate de l'interface de cloisonnement, et de telle façon à autoriser lors d'une maintenance sur le système de traitement, le retrait de la partie mobile de la machine de traitement alors que ladite interface de cloisonnement de la machine de traitement obture toujours l'ouverture du mur, l'ouverture de l'interface de cloisonnement alors obturée par le deuxième système de porte.

Selon des caractéristiques optionnelles de l'invention, prises seules ou en combinaison :
- la partie mobile embarque la cuve de traitement, un système de joint solidaire de la partie mobile, et/ou solidaire de l'interface de cloisonnement assurant une étanchéité entre la seconde ouverture de la cuve et l'ouverture correspondante de l'interface de cloisonnement dans la position de fonctionnement de la machine de traitement pour laquelle la deuxième ouverture est plaquée sur l'interface de cloisonnement, par l'intermédiaire dudit système de joint, et autorisant le retrait de la partie mobile lorsque retirée, ou alternativement ;
- la cuve de traitement est solidaire de l'interface de cloisonnement, un système de connexion hydraulique permettant de connecter les tuyaux du ou des circuits du système de traitement embarqué(s) à la partie mobile, avec les tuyaux du ou des circuits de la cuve de traitement, dans ladite position d'utilisation de la machine de traitement, et de les déconnecter lors du retrait de la partie mobile ;
- l'interface de cloisonnement comprend une cloison destinée à s'étendre le long de l'ouverture du mur en l'obturant, ainsi qu'un système de fixation périphérique solidaire de la cloison, destiné à s'étendre le long du chant de l'ouverture du mur, le long de ses montants, voire de son bord supérieur ;
- le système de fixation périphérique comprend, pour chaque montant de l'ouverture, un premier élément, dit d'appui destiné à appuyer d'un côté du mur, un second élément dit de contre appui destiné à appuyer de l'autre côté du mur, ainsi qu'un système de réglage et/ou serrage, par exemple à vis, configuré de manière à ajuster l'écartement entre le premier élément et le second élément à l'épaisseur du mur, voire permettre de serrer le mur entre le premier élément et le second élément ;
- l'interface de cloisonnement comprend un cadre périphérique, à fonction de cache, destiné à s'étendre à partir de la face de l'interface de cloisonnement contre laquelle est destiné à venir se positionner le châssis de la partie mobile en position d'utilisation le long des chants de l'ouverture du mur en les recouvrant, puis en s'étendant en saillie au-delà de la face du mur côté partie mobile, les parois intérieures du cadre formant un logement pour la partie amovible, de dimension en largeur ajustée à la dimension de la partie mobile ;
- le châssis autoportant de la partie mobile est un mobilier destiné à être en appui sur le sol dans ladite position d'utilisation ;
- le châssis autoportant de la partie mobile comporte des organes de roulement, tels que des roulettes en encore des roues omnidirectionnelles à sa partie inférieure, de préférence verrouillable, autorisant le retrait de la partie amovible de la machine en la faisant rouler au sol.

L'invention concerne encore un ensemble comprenant une machine de traitement selon l'invention, et un mur séparant une première enceinte à atmosphère contrôlée/non contrôlée d'une seconde enceinte à atmosphère non contrôlée/contrôlée, présentant une ouverture traversante, et dans lequel ladite interface de cloisonnement est fixée à demeure au mur configurée de manière à obturer l'ouverture du mur, et de telle façon à autoriser lors d'une maintenance sur le système de traitement, le retrait de la partie mobile de la machine de traitement alors que ladite interface de cloisonnement de la machine de traitement obture toujours l'ouverture du mur, l'ouverture de l'interface de cloisonnement alors obturée par le deuxième système de porte.

L'invention concerne encore un procédé de maintenance d'une machine de traitement d'un ensemble selon l'invention, dans lequel on procède à la maintenance du système de traitement par la mise en œuvre des étapes suivantes :
- retrait de la partie mobile de la machine de traitement alors que ladite interface de cloisonnement de la machine de traitement obture toujours l'ouverture du mur, ladite ouverture de l'interface de cloisonnement alors obturée par le deuxième système de porte,
- transport de la partie mobile vers un atelier de maintenance distant et révision/réparation dudit système de traitement,
- retour de la partie mobile révisée/réparée et mise en place dans sa position d'utilisation à proximité immédiate de l'interface de cloisonnement.

Selon un second principe général, l'invention concerne encore un ensemble comportant une machine de traitement d'un appareil médical, tel qu'un laveur désinfecteur d'endoscope, et un mur de séparation, ladite machine s'intégrant sur un mur, dit mur de séparation au niveau d'une ouverture traversante du mur de séparation en l'obturant, le mur séparant une première enceinte à atmosphère contrôlée/non contrôlée d'une seconde enceinte à atmosphère non contrôlée/contrôlée,
ladite machine comportant sur un même châssis autoportant:
- une cuve de traitement présentant une première ouverture configurée de manière à permettre le chargement/déchargement d'un appareil médical à partir de la première enceinte et une seconde ouverture configurée de manière à permettre le chargement/déchargement d'un appareil médical à partir de la seconde enceinte,
- un premier système de porte assurant la fermeture de manière étanche de la première ouverture,
- un deuxième système de porte assurant la fermeture de manière étanche de la seconde ouverture,
- un système de traitement d'une appareil médical présentant un ou plusieurs circuits de traitement, y compris une ou plusieurs pompes, une ou plusieurs électrovalves, permettant le traitement des surfaces intérieures et extérieures de l'appareil médical contenu dans la cuve de traitement par immersion et/ou par aspersion, à partir d'une solution désinfectante prélevée dans un réservoir hors de la cuve de traitement,
- un système automate programmable assurant la commande du système de traitement, en particulier du moteur et des électrovalves du système, configuré pour la mise en œuvre d'un cycle de désinfection d'un appareil médical.

Selon l'invention, ledit ensemble comporte un système de porte, articulé au mur qui est configuré pour passer d'une première position d'ouverture pour laquelle ladite porte libère l'ouverture du mur dans la position d'utilisation de la machine pour laquelle le châssis autoportant de la machine est positionné contre l'ouverture du mur en l'obturant vers une deuxième position de fermeture autorisant l'obturation de l'ouverture du mur par le système du mur après le retrait de la machine de traitement.

L'invention sera mieux comprise à la lecture de la description accompagnée des dessins en annexe parmi lesquels :
- La figure 1 est une vue d'un ensemble selon un premier mode de réalisation comprenant une machine de traitement selon l'invention, illustrant plus particulièrement la partie mobile de la machine qui embarque la cuve de traitement, dans une position retirée et écartée de l'interface de cloisonnement, elle-même fixée à demeure au mur de séparation.
- La figure 2 est vue de l'ensemble selon la figure 1 dans la position d'utilisation de la machine pour laquelle la partie mobile est plaquée contre l'interface de cloisonnement,
- La figure 3 est une vue schématique de coupe selon un plan horizontal de la machine en position d'utilisation du premier mode de réalisation dudit ensemble,
- La figure 4 est une vue schématique de coupe selon un plan vertical de la machine en position d'utilisation, illustrant plus particulièrement le système de joint assurant l'étanchéité entre la deuxième ouverture de la cuve et l'ouverture de l'interface de cloisonnement dans ladite position d'utilisation de la machine, le système de joint autorisant la séparation de ces deux parties lors du retrait de la partie mobile,
- La figure 5 et la figure 6 sont deux vues, de part et d'autre du mur de séparation, d'une machine en position d'utilisation, ladite partie mobile plaquée contre l'interface de cloisonnement dans une position où le système d'étanchéité assure l'étanchéité entre la cuve et l'interface de cloisonnement,
- La figure 7 est une vue, selon une coupe horizontale, d'un mode de réalisation du système de fixation périphérique de l'interface de cloisonnement, avec un exemple de système de réglage permettant d'ajuster le montage à l'épaisseur du mur,
- La figure 8 est une vue d'une machine selon un second mode de réalisation pour laquelle la cuve de traitement est solidaire de l'interface de cloisonnement, elle-même fixée à demeure au mur.
- Les figures 9 et 10 sont deux vues schématiques d'un troisième mode de réalisation pour laquelle un système de porte ouvre une ouverture d'un mur lorsque la machine de traitement est en position d'utilisation dans cette ouverture du mur, et permet de fermer cette ouverture lorsque la machine de traitement est retirée pour maintenance.

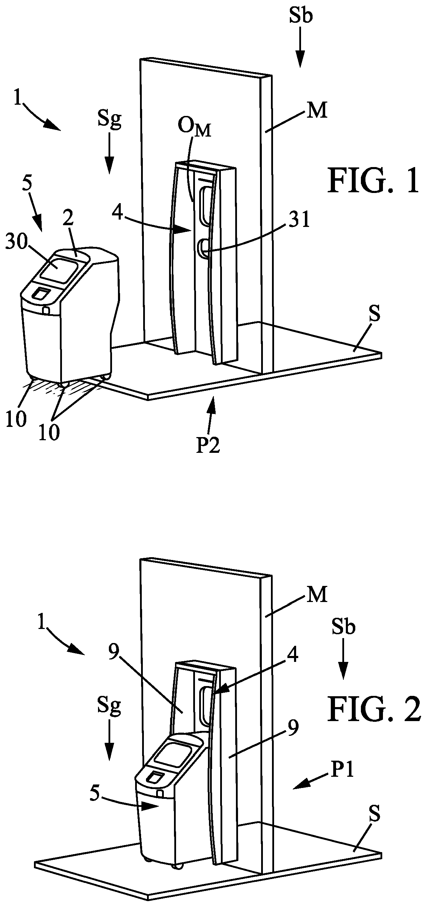

Aussi l'invention concerne tout d'abord, et selon un premier principe général illustré non limitativement des figures 1 à 8, une machine de traitement 1 d'un appareil médical, tel qu'un laveur désinfecteur d'endoscope, destiné à être intégrer sur un mur M, dit mur de séparation au niveau d'une ouverture traversante O_{M} du mur de séparation.

Ce mur étant destiné à séparer une première enceinte Sg à atmosphère contrôlée/non contrôlée, telle qu'une salle grise, d'une seconde enceinte Sb à atmosphère non contrôlée/contrôlée, telle qu'une salle blanche.

Cette machine de traitement comporte :
- une cuve de traitement 2 présentant une première ouverture 20 configurée de manière à permettre le chargement/déchargement d'un appareil médical à partir de la première enceinte Sg et une seconde ouverture 21 configurée de manière à permettre le chargement/déchargement d'un appareil médical à partir de la seconde enceinte Sb,
- un premier système de porte 30 assurant la fermeture de manière étanche de la première ouverture 20,
- un deuxième système de porte 31 assurant la fermeture de manière étanche de la seconde ouverture 21,
- un système de traitement d'un appareil médical présentant un ou plusieurs circuits de traitement, y compris une ou plusieurs pompes, une ou plusieurs électrovalves, permettant le traitement des surfaces intérieures et extérieures de l'appareil médical contenu dans la cuve de traitement 2 par immersion et/ou par aspersion, à partir d'une solution désinfectante prélevée dans un réservoir tel qu'un bidon positionné hors de la cuve de traitement,
- un système automate programmable assurant la commande du système de traitement, en particulier du moteur et des électrovalves du système, configuré pour la mise en œuvre d'un cycle de désinfection d'un appareil médical.

En position d'utilisation de la machine de traitement, il est ainsi possible de charger la cuve de traitement à partir de la seconde enceinte telle qu'une salle blanche d'un bloc opératoire avec un appareil médical, au travers de la deuxième ouverture, puis de fermer le second système de porte. On procède à la désinfection de l'appareil contenu dans la cuve, le premier système de porte 30 et le second système de porte 31 alors en position de fermeture. Le retrait de l'appareil désinfecté s'effectue, après ouverture du premier système de porte à partir de la première enceinte telle qu'une sille grise. Il est également envisageable de procéder suivant une démarche inverse, en chargeant l'endoscope souillé depuis l'enceinte à atmosphère non contrôlée (la salle grise), et de le décharger côté enceinte à atmosphère contrôlée (la salle blanche).

Selon un mode de réalisation de l'invention, ladite machine 1 comprend de manière notable, une interface 4 de cloisonnement destinée à être fixée à demeure au mur, et une partie mobile 5, avantageusement amovible de l'interface de cloisonnement lors de la maintenance.

On entend ici par fixé « *à demeure* » le fait que l'interface de cloisonnement n'est pas retirée lors de la maintenance. Cette interface 4 de cloisonnement est en effet destinée à être fixée à demeure au mur M et configurée de manière à toujours obturer l'ouverture O_{M} du mur.

Ladite interface de cloisonnement 4 comporte au moins une ouverture traversante 40 destinée à être en regard de la deuxième ouverture 21 de la cuve 2, au moins en position de fonctionnement P1 de la machine de traitement.

De manière notable, le deuxième système de porte 31 est solidaire de ladite interface de cloisonnement 4 (et non de la partie mobile qui est définie ci-après), assurant dans sa position de fermeture l'obturation de ladite ouverture 40 de l'interface de cloisonnement.

La partie mobile 5, est avantageusement amovible de l'interface de cloisonnement 4, et comporte un châssis autoportant, embarquant au moins en partie la ou les pompes et les électrovalves dudit système de traitement de l'appareil médical ainsi que ledit automate programmable. Autrement dit, cette partie mobile 5 embarque les systèmes électriques et hydrauliques de la machine requérant typiquement une maintenance.

Cette partie mobile 5 est configurée pour se positionner à proximité immédiate de l'interface de cloisonnement 4, en position de fonctionnement P1 de la machine de traitement (visible à la figure 2), et peut être retirée dans une position P2 (visible à la figure 1).

Avantageusement, il est ainsi possible, lors d'une maintenance sur le système de traitement, de retirer la partie mobile 5 de la machine de traitement alors que ladite interface de cloisonnement 4 de la machine de traitement obture toujours l'ouverture du mur, l'ouverture 40 de l'interface de cloisonnement 4 alors obturée par le deuxième système de porte 31.

Il est ainsi toujours possible de conserver une étanchéité de séparation entre la première enceinte Sg telle que la salle grise, et la seconde enceinte telle que la salle blanche, compatible à la mise en œuvre d'un contrôle de la qualité de l'air dans l'une des enceintes, à savoir celle à atmosphère contrôlée.

La salle blanche Sb peut être typiquement à atmosphère contrôlée par purification de l'air (par exemple par filtration HEPA) et mise en surppression de l'enceinte : le fait de conserver une bonne étanchéité du mur de séparation (même après retrait de la partie mobile 5) permet de garantir la qualité de l'atmosphère, même en cas de maintenance lourde.

L'invention concerne encore un ensemble comprenant une machine de traitement 1 selon l'invention, et un mur M séparant une première enceinte Sg à atmosphère contrôlée/non contrôlée d'une seconde enceinte Sb à atmosphère non contrôlée/contrôlée, présentant une ouverture traversante O_{M}. Dans un tel ensemble, ladite interface de cloisonnement 4 est fixée à demeure au mur et configurée de manière à obturer l'ouverture du mur, et de telle façon à autoriser lors d'une maintenance sur le système de traitement, le retrait de la partie mobile 5 de la machine de traitement 1 alors que ladite interface de cloisonnement 4 de la machine de traitement obture toujours l'ouverture O_{M} du mur, l'ouverture 40 de l'interface de cloisonnement alors obturée par le deuxième système de porte 31.

L'invention concerne encore un procédé de maintenance d'une machine de traitement 1 d'un ensemble selon l'invention, dans lequel on procède à la maintenance du système de traitement par la mise en œuvre des étapes suivantes :
- retrait de la partie mobile 5 de la machine de traitement alors que ladite interface de cloisonnement 4 de la machine de traitement obture toujours l'ouverture Om du mur, ladite ouverture 40 de l'interface de cloisonnement alors obturée par le deuxième système de porte 31,
- transport de la partie mobile vers un atelier de maintenance distant et révision/réparation dudit système de traitement,
- retour de la partie mobile 5 révisée/réparée et mise en place dans sa position d'utilisation P1 à proximité immédiate de l'interface de cloisonnement 4.

Eventuellement, et pendant que la partie mobile 5 retirée est en révision en atelier, il est possible de mettre en place une autre partie mobile 5 (identique et de même nature) dans la position d'utilisation. Cette autre partie mobile ferait par exemple l'objet d'un prêt par le prestataire de la révision/réparation, et afin de conserver les capacités de désinfection le temps de la maintenance.

Selon un mode de réalisation, la partie mobile 5 embarque la cuve de traitement 2, un système de joint 7 solidaire de la partie mobile 5, et/ou solidaire de l'interface de cloisonnement 4 assurant une étanchéité entre la seconde ouverture 21 de la cuve 2 et l'ouverture 40 correspondante de l'interface de cloisonnement 4 dans la position de fonctionnement P1 de la machine de traitement. Dans cette position P1, la deuxième ouverture 21 de la cuve 2 est plaquée sur l'interface de cloisonnement 4, par l'intermédiaire dudit système de joint 7. Ce système de joint 7 autorise encore le retrait de la partie mobile 5 lorsqu'extraite.

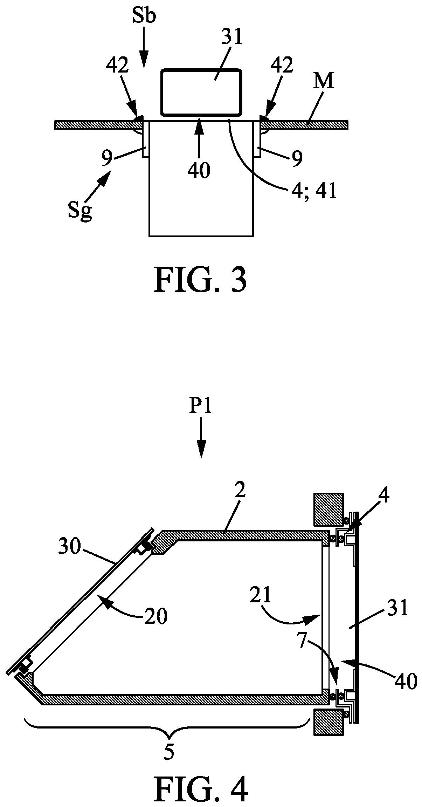

Un tel mode de réalisation est illustré à titre indicatif aux figures 1 à 7, et plus particulièrement à la vue de la figure 4. Comme illustré schématiquement à la figure 4, dans la position d'utilisation P1, la seconde ouverture de la cuve est plaquée contre l'ouverture 40 de l'interface de cloisonnement 4, par l'intermédiaire du système de joint 7. Dans cette position P1, le système de joint alors comprimé assure l'étanchéité entre l'interface de cloisonnement et la seconde ouverture de la cloison, en permettant au deuxième système de porte 31 d'obturer la seconde ouverture 21. Lorsque la partie mobile 5 est retirée, le système de joint 7 n'est plus comprimé, au moins partiellement.

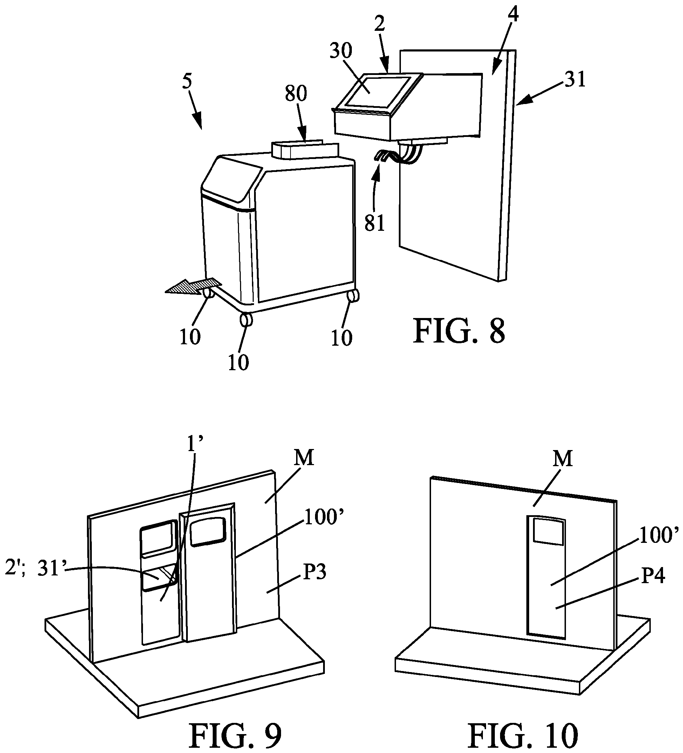

Selon un autre mode de réalisation (alternatif), illustré à titre indicatif à la figure 8, la cuve de traitement 2 est solidaire de l'interface de cloisonnement 4, un système de connexion 80, 81 hydraulique permettant de connecter les tuyaux du ou des circuits du système de traitement embarqué à la partie mobile 5, avec les tuyaux du ou des circuits de la cuve de traitement 2, dans ladite position P1 d'utilisation de la machine de traitement, et de les déconnecter lors du retrait de la partie mobile 5. Ce mode de réalisation se distingue du précédent en ce que la cuve de traitement (ainsi que le premier et second système de porte) est solidaire de l'interface de cloisonnement 4, et non de la partie mobile.

Le système de connexion 80, 81 est alors nécessaire pour connecter les conduites du ou des circuits embarqués à la partie mobile 5, avec les conduites correspondantes de la cuve de traitement 2, et les déconnecter pour autoriser le retrait de la partie mobile 5.

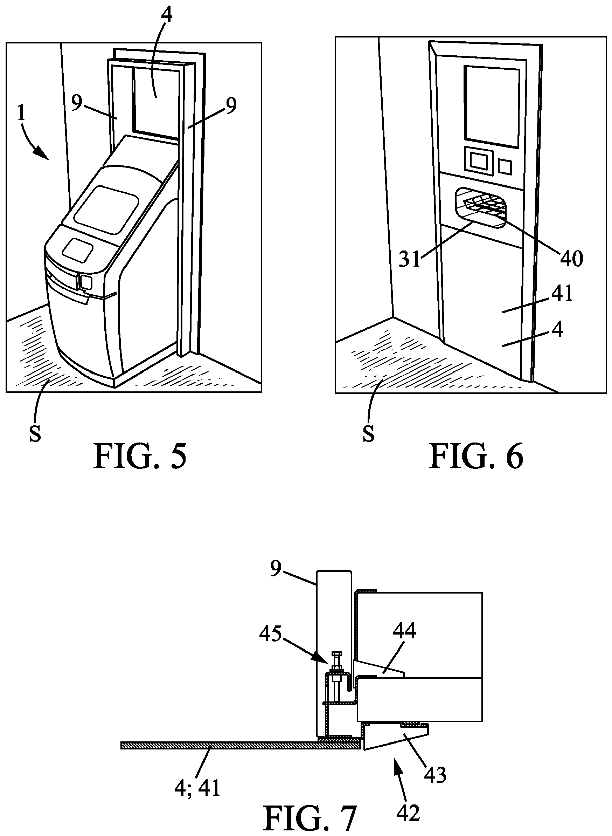

Selon un mode de réalisation illustré à titre indicatif à la figure 3 ou à la figure 7, l'interface de cloisonnement 4 comprend une cloison 41 destinée à s'étendre le long de l'ouverture du mur en l'obturant, de manière sensiblement parallèle au mur, ainsi qu'un système 42 de fixation périphérique solidaire de la cloison, s'étendant le long du chant de l'ouverture du mur, le long de ses montants, voire de son bord supérieur.

Ce système de fixation périphérique 42 peut comprendre, pour chaque montant de l'ouverture O_{M} :
- un premier élément 43, dit d'appui tel qu'un profilé destiné à appuyer d'un côté du mur,
- un second élément 44 dit de contre appui, tel qu'un profilé destiné à appuyer de l'autre côté du mur,
- un système 45 de réglage et/ou serrage, par exemple à vis, configuré de manière à ajuster l'écartement entre le premier élément 43 d'appui et le second élément 44 de contre appui à l'épaisseur du mur M, voire permettre de serrer le mur entre le premier élément d'appui et le second élément de contre appui.

La figure 7 illustre à titre d'indicatif un système 45 de réglage et de serrage, à vis, qui permet de rapprocher par vissage le premier élément d'appui 43 vers le deuxième élément 44 de contre appui, ainsi de serrer l'épaisseur du mur.

Selon un mode de réalisation, l'interface de cloisonnement 4 peut comprendre un cadre périphérique 9, à fonction de cache, destiné à s'étendre à partir de la face de l'interface de cloisonnement 4 contre laquelle est destiné à venir se positionner le châssis de la partie mobile 5 en position d'utilisation P1, en largeur le long des chants de l'ouverture du mur M en les recouvrant, voire en s'étendant en saillie au-delà de la face du mur côté partie mobile 5.

La fonction de ce cadre est esthétique, à savoir de cacher les jours présents, de part et d'autre de la partie mobile, entre chaque paroi latérale du mobilier et le chant de l'ouverture correspondant. Avantageusement, les parois intérieures du cadre 9 forment un logement pour la partie mobile 5, de dimension en largeur ajustée à la dimension de la partie mobile 5 alors que les parois extérieures du cadre 9 affleurent les chants de l'ouverture du mur.

Selon un mode de réalisation, le châssis autoportant de la partie mobile 5 est un mobilier destiné à être en appui sur le sol S dans ladite position d'utilisation P1. Le châssis autoportant de la partie mobile 5 peut comporter des organes de roulement 10, tels que des roulettes en encore des roues omnidirectionnelles à sa partie inférieure, de préférence verrouillable, autorisant le retrait de la partie mobile 5 de la machine en la faisant rouler au sol S, ou inversement autorisant sa mise en place contre l'interface de cloisonnement.

Les figures 9 et 10 divulguent un deuxième principe général qui permet de répondre au problème posé par l'invention.

L'ensemble illustré aux figures 9 et 10 comportant une machine de traitement d'un appareil médical, tel qu'un laveur désinfecteur d'endoscope, et un mur de séparation M, ladite machine s'intégrant sur un mur, dit mur de séparation au niveau d'une ouverture traversante du mur de séparation en l'obturant, le mur séparant une première enceinte Sg à atmosphère contrôlée/non contrôlée d'une seconde enceinte à atmosphère Sb non contrôlée/contrôlée,

La machine est d'un seul tenant et comporte sur un même châssis autoportant:
- une cuve de traitement présentant une première ouverture configurée de manière à permettre le chargement/déchargement d'un appareil médical à partir de la première enceinte et une seconde ouverture configurée de manière à permettre le chargement/déchargement d'un appareil médical à partir de la seconde enceinte,
- un premier système de porte assurant la fermeture de manière étanche de la première ouverture,
- un deuxième système de porte assurant la fermeture de manière étanche de la seconde ouverture,
- un système de traitement d'un appareil médical présentant un ou plusieurs circuits de traitement, y compris une ou plusieurs pompes, une ou plusieurs électrovalves, permettant le traitement des surfaces intérieures et extérieures de l'appareil médical contenu dans la cuve de traitement par immersion et/ou par aspersion, à partir d'une solution désinfectante prélevée dans un réservoir hors de la cuve de traitement,
- un système automate programmable assurant la commande du système de traitement, en particulier du moteur et des électrovalves du système, configuré pour la mise en œuvre d'un cycle de désinfection d'un appareil médical.

Selon ce second principe général, ledit ensemble comporte un système de porte 100, articulé (par exemple par charnière ou glissières) au mur qui est configuré pour passer d'une première position d'ouverture P3 pour laquelle ladite porte libère l'ouverture du mur M dans la position d'utilisation de la machine 1' pour laquelle le châssis autoportant de la machine est positionné contre l'ouverture du mur en l'obturant vers une deuxième position de fermeture P4 autorisant l'obturation de l'ouverture du mur par le système de porte 100 après le retrait de la machine de traitement.

Le principe illustré aux figures 9 et 10 nécessite, après retrait de la machine 1' pour raison de maintenance, de fermer le système de porte 100' pour obturer l'ouverture du mur laissée alors béante par le retrait de la machine 1', ce qui est moins satisfaisant en terme de sécurité que le premier principe général des figures 1 à 8, qui repose sur l'utilisation d'une machine comportant une interface de cloisonnement fixée à demeure au mur et configurée de manière à toujours obturer cette ouverture O_{M}.

### NOMENCLATURE

### Principe des figures 1 à 8 :

1. Machine de traitement,
2. Cuve de traitement,
20, 21. Première et seconde ouverture de la cuve,
30, 31. Premier système de porte et deuxième système de porte,
4. Interface de cloisonnement,
40. Ouverture de l'interface de cloisonnement destinée à être positionnée en regard de la seconde ouverture de la cuve,
41. Cloison,
42. Système de fixation périphérique,
43. 44. Premier élément d'appui et second élément de contre appui
45. Système de réglage et de serrage,
5. Partie mobile, amovible par rapport à l'interface de cloisonnement,
7. Système de joint entre la cuve et l'interface de cloisonnement (mode de réalisation des Figure 1 à 7),
9. Cadre
10. Organes de roulement
80,81. Système de connexion hydraulique entre les conduites de la cuve de traitement, solidaire de l'interface de cloisonnement et les tuyaux du système de traitement embarqué à la partie mobile (mode de réalisation de la figure 8)
M. Mur de séparation,
O_{M..} Ouverture traversante (Mur)
Sg, Sb. Première enceinte telle que salle grise, et deuxième enceinte telle que salle blanche séparée par le mur,
S. Sol.
P1. Position de fonctionnement de la machine pour laquelle la partie mobile jouxte l'interface de cloisonnement et sont reliés (physiquement ou hydrauliquement),
P2. Position de maintenance de la machine pour laquelle la partie mobile est retirée.
Principe des figures 9 et 10.
1'. Machine de traitement,
M'. Mur de séparation,
Sg', Sb'. Première enceinte telle que salle grise, et deuxième enceinte telle que salle blanche séparée par le mur,
100'. Système de porte
P3. Première position d'ouverture du système de porte,
P4. Deuxième position de fermeture du système de porte.

## Revendications

1. Machine de traitement (1) d'un appareil médical, tel qu'un laveur désinfecteur d'endoscope, destiné à être intégrer sur un mur (M), dit mur de séparation au niveau d'une ouverture traversante (O_{M}) du mur de séparation, le mur étant destiné à séparer une première enceinte (Sg) à atmosphère contrôlée/non contrôlée d'une seconde enceinte (Sb) à atmosphère non contrôlée/contrôlée, ladite machine comportant :
- une cuve de traitement (2) présentant une première ouverture (20) configurée de manière à permettre le chargement/déchargement d'un appareil médical à partir de la première enceinte (Sg) et une seconde ouverture (21) configurée de manière à permettre le chargement/déchargement d'un appareil médical à partir de la seconde enceinte (Sb),
- un premier système de porte (30) assurant la fermeture de manière étanche de la première ouverture (20),
- un deuxième système de porte (31) assurant la fermeture de manière étanche de la seconde ouverture (21),
- un système de traitement d'un appareil médical présentant un ou plusieurs circuits de traitement, y compris une ou plusieurs pompes, une ou plusieurs électrovalves, permettant le traitement des surfaces intérieures et extérieures de l'appareil médical contenu dans la cuve de traitement (2) par immersion et/ou par aspersion, à partir d'une solution désinfectante prélevée dans un réservoir hors de la cuve de traitement,
- un système automate programmable assurant la commande du système de traitement, en particulier du moteur et des électrovalves du système, configuré pour la mise en œuvre d'un cycle de désinfection d'un appareil médical,
**caractérisée en ce que** ladite machine (1) comprend :
- une interface (4) de cloisonnement destinée à être fixée à demeure au mur (M) et configurée de manière à obturer l'ouverture (O_{M}) du mur, ladite interface de cloisonnement comportant au moins une ouverture traversant (40) destinée à être en regard de la deuxième ouverture de la cuve, au moins en position de fonctionnement (P1) de la machine de traitement, le deuxième système de porte (31) étant solidaire de ladite interface de cloisonnement (4), assurant dans sa position de fermeture l'obturation de ladite ouverture (40) de l'interface de cloisonnement,
- une partie mobile (5), amovible de l'interface de cloisonnement (4) comportant un châssis autoportant, et embarquant au moins en partie la ou les pompes et les électrovalves dudit système de traitement de l'appareil médical ainsi que ledit automate programmable, qui en position de fonctionnement (P1) de la machine de traitement est configuré pour se positionner à proximité immédiate de l'interface de cloisonnement (4),
et de telle façon à autoriser lors d'une maintenance sur le système de traitement, le retrait de la partie mobile (5) de la machine de traitement alors que ladite interface de cloisonnement (4) de la machine de traitement obture toujours l'ouverture du mur, l'ouverture (40) de l'interface de cloisonnement (4) alors obturée par le deuxième système de porte (31).

2. Machine selon la revendication 1, dans laquelle la partie mobile (5) embarque la cuve de traitement (2), un système de joint (7) solidaire de la partie mobile (5), et/ou solidaire de l'interface de cloisonnement (4) assurant une étanchéité entre la seconde ouverture (21) de la cuve et l'ouverture correspondante de l'interface de cloisonnement dans la position de fonctionnement (P1) de la machine de traitement pour laquelle la deuxième ouverture (21) est plaquée sur l'interface de cloisonnement, par l'intermédiaire dudit système de joint, et autorisant le retrait de la partie mobile lorsque retirée.

3. Machine selon la revendication 1, dans laquelle la cuve de traitement (2) est solidaire de l'interface de cloisonnement (4), un système de connexion (80, 81) hydraulique permettant de connecter les tuyaux du ou des circuits du système de traitement embarqué à la partie mobile (5), avec les tuyaux du ou des circuits de la cuve de traitement, dans ladite position (P1) d'utilisation de la machine de traitement, et de les déconnecter lors du retrait de la partie mobile (5)

4. Machine selon l'une des revendications 1 à 3, dans laquelle l'interface de cloisonnement (4) comprend une cloison (41) destinée à s'étendre le long de l'ouverture du mur en l'obturant, ainsi qu'un système (42) de fixation périphérique solidaire de la cloison, s'étendant le long du chant de l'ouverture du mur, le long de ses montants, voire de son bord supérieur.

5. Machine selon l'une des revendications 1 à 4, dans lequel le système de fixation périphérique (42) comprend :
- un premier élément (43), dit d'appui destiné à appuyer d'un côté du mur,
- un second élément (44) dit de contre appui destiné à appuyer de l'autre côté du mur,
- un système (45) de réglage et/ou serrage, par exemple à vis, configuré de manière à ajuster l'écartement entre le premier élément (43) et le second élément (44) à l'épaisseur du mur (M), voire permettre de serrer le mur entre le premier élément et le second élément.

6. Machine selon la revendication 4 ou 5, dans lequel l'interface de cloisonnement (4) comprend un cadre périphérique (9), à fonction de cache, destiné à s'étendre à partir de la face de l'interface de cloisonnement contre laquelle est destiné à venir se positionner le châssis de la partie mobile (5) en position d'utilisation P1 ; le long des chants de l'ouverture du mur (M) en les recouvrant, voire en s'étendant en saillie au-delà de la face du mur côté partie mobile (5), les parois intérieures du cadre (9) formant un logement pour la partie amovible, de dimension en largeur ajustée à la dimension de la partie mobile (5).

7. Machine selon l'une des revendications 1 à 6, dans lequel le châssis autoportant de la partie mobile (5) est un mobilier destiné à être en appui sur le sol (S) dans ladite position d'utilisation (P1).

8. Machine selon la revendication 7, dans lequel le châssis autoportant de la partie mobile (5) comporte des organes de roulement (10), telles que des roulettes en encore des roues omnidirectionnelles à sa partie inférieure, de préférence verrouillable, autorisant le retrait de la partie mobile (5) de la machine en la faisant rouler au sol (S).

9. Ensemble comprenant une machine de traitement (1) selon l'une des revendications 1 à 8, et un mur (M) séparant une première enceinte (Sg) à atmosphère contrôlée/non contrôlée d'une seconde enceinte (Sb) à atmosphère non contrôlée/contrôlée, présentant une ouverture traversante (O_{M}), et dans lequel ladite interface de cloisonnement est fixée à demeure au mur configurée de manière à obturer l'ouverture du mur, et de telle façon à autoriser lors d'une maintenance sur le système de traitement, le retrait de la partie mobile (5) de la machine de traitement alors que ladite interface de cloisonnement (4) de la machine de traitement obture toujours l'ouverture (O_{M}) du mur, l'ouverture (40) de l'interface de cloisonnement alors obturée par le deuxième système de porte (31).

10. Procédé de maintenance d'une machine de traitement (1) d'un ensemble selon la revendication 9, dans lequel on procède à la maintenance du système de traitement par la mise en œuvre des étapes suivantes :
- retrait de la partie mobile (5) de la machine de traitement alors que ladite interface de cloisonnement (4) de la machine de traitement obture toujours l'ouverture (Om) du mur, ladite ouverture (40) de l'interface de cloisonnement alors obturée par le deuxième système de porte (31),
- transport de la partie mobile vers un atelier de maintenance distant et révision/réparation dudit système de traitement,
- retour de la partie mobile (5) révisée/réparée et mise en place dans sa position d'utilisation (P1) à proximité immédiate de l'interface de cloisonnement (4).

11. Ensemble comportant une machine de traitement (1') d'un appareil médical, tel qu'un laveur désinfecteur d'endoscope, et un mur de séparation, ladite machine s'intégrant sur un mur, dit mur de séparation au niveau d'une ouverture traversante du mur de séparation en l'obturant, le mur séparant une première enceinte (Sg) à atmosphère contrôlée/non contrôlée d'une seconde enceinte à atmosphère (Sb) non contrôlée/contrôlée,
ladite machine comportant sur un même châssis autoportant:
- une cuve de traitement (2') présentant une première ouverture configurée de manière à permettre le chargement/déchargement d'un appareil médical à partir de la première enceinte et une seconde ouverture configurée de manière à permettre le chargement/déchargement d'un appareil médical à partir de la seconde enceinte,
- un premier système de porte assurant la fermeture de manière étanche de la première ouverture,
- un deuxième système de porte (31') assurant la fermeture de manière étanche de la seconde ouverture,
- un système de traitement d'un appareil médical présentant un ou plusieurs circuits de traitement, y compris une ou plusieurs pompes, une ou plusieurs électrovalves, permettant le traitement des surfaces intérieures et extérieures de l'appareil médical contenu dans la cuve de traitement par immersion et/ou par aspersion, à partir d'une solution désinfectante prélevée dans un réservoir hors de la cuve de traitement,
- un système automate programmable assurant la commande du système de traitement, en particulier du moteur et des électrovalves du système, configuré pour la mise en œuvre d'un cycle de désinfection d'un appareil médical,
**caractérisé en ce que** ledit ensemble comporte un système de porte (100), articulé au mur
qui est configuré pour passer d'une première position d'ouverture (P3) pour laquelle ladite porte libère l'ouverture du mur (M) dans la position d'utilisation de la machine pour laquelle le châssis autoportant de la machine est positionné contre l'ouverture du mur en l'obturant vers une deuxième position de fermeture (P4) autorisant l'obturation de l'ouverture du mur par le système du mur après le retrait de la machine de traitement.

## Patentansprüche

1. Behandlungsmaschine (1) für ein medizinisches Gerät, wie zum Beispiel ein Endoskop-Reinigungs- und Desinfektionsgerät, die dazu bestimmt ist, in eine Wand (M), bezeichnet als Trennwand, in Höhe einer Durchgangsöffnung (O_{M}) der Trennwand integriert zu werden, wobei die Wand dazu bestimmt ist, ein erstes Gehäuse (Sg) mit einer geregelten/nicht geregelten Atmosphäre von einem zweiten Gehäuse (Sb) mit einer geregelten/nicht geregelten Atmosphäre zu trennen, wobei die Maschine Folgendes umfasst:
- einen Behandlungstank (2) mit einer ersten Öffnung (20), die so konfiguriert ist, dass sie das Be-/Entladen eines medizinischen Geräts aus dem ersten Gehäuse (Sg) ermöglicht, und einer zweiten Öffnung (21), die so konfiguriert ist, dass sie das Be-/Entladen eines medizinischen Geräts aus dem zweiten Gehäuse (Sb) ermöglicht,
- ein erstes Türsystem (30), das die Abdichtung der ersten Öffnung (20) sicherstellt,
- ein zweites Türsystem (31), das die Abdichtung der zweiten Öffnung (21) sicherstellt,
- ein Behandlungssystem für ein medizinisches Gerät mit einem oder mehreren Behandlungskreisläufen, einschließlich einer oder mehrerer Pumpen, einem oder mehreren Magnetventilen, zur Behandlung der Innen- und Außenflächen des in dem Behandlungstank (2) enthaltenen medizinischen Geräts durch Eintauchen und/oder Besprühen mittels einer Desinfektionslösung, die aus einem Tank außerhalb des Behandlungstanks entnommen wird,
- ein programmierbares Automatensystem zur Steuerung des Behandlungssystems, insbesondere des Motors und der Magnetventile des Systems, das für die Durchführung eines Desinfektionszyklus eines medizinischen Geräts konfiguriert ist, **dadurch gekennzeichnet, dass** die Maschine (1) Folgendes umfasst:
- eine Trennwandschnittstelle (4), die zur dauerhaften Befestigung an der Wand (M) bestimmt ist und so konfiguriert ist, dass sie die Öffnung (O_{M}) der Wand verschließt, wobei die Trennwandschnittstelle mindestens eine Durchgangsöffnung (40) aufweist, die dazu bestimmt ist, mindestens in der Betriebsstellung (P1) der Behandlungsmaschine gegenüber der zweiten Öffnung des Behälters angeordnet zu sein, wobei das zweite Türsystem (31) fest mit der Trennwandschnittstelle (4) verbunden ist und in seiner Schließstellung den Verschluss der Öffnung (40) der Trennwandschnittstelle sicherstellt,
- einen beweglichen Teil (5), der von der Trennwandschnittstelle (4) abnehmbar ist, umfassend einen selbsttragenden Rahmen, und der mindestens teilweise die Pumpe(n) und die Elektroventile des Behandlungssystems des medizinischen Geräts sowie den programmierbaren Automaten enthält, der in der Betriebsposition (P1) der Behandlungsmaschine so konfiguriert ist, dass er in unmittelbarer Nähe der Trennwandschnittstelle (4) positioniert werden kann, und zwar derart, dass während der Wartung des Behandlungssystems das Entfernen des beweglichen Teils (5) der Behandlungsmaschine möglich ist, während die Trennschnittstelle (4) der Behandlungsmaschine weiterhin die Öffnung der Wand verschließt, wobei die Öffnung (40) der Trennschnittstelle (4) dann durch das zweite Türsystem (31) verschlossen wird.

2. Maschine nach Anspruch 1, wobei der bewegliche Teil (5) den Behandlungstank (2), ein mit dem beweglichen Teil (5) fest verbundenes Dichtungssystem (7), trägt und/oder fest mit der Trennwandschnittstelle (4) verbunden ist, die eine Abdichtung zwischen der zweiten Öffnung (21) des Tanks und der entsprechenden Öffnung der Trennwandschnittstelle in der Betriebsposition (P1) der Behandlungsmaschine gewährleistet, wofür die zweite Öffnung (21) mittels des Dichtungssystems gegen die Trennwandschnittstelle gedrückt wird, und die das Entfernen des beweglichen Teils ermöglicht, wenn es entfernt wird.

3. Maschine nach Anspruch 1, wobei der Behandlungstank (2) fest mit der Trennwandschnittstelle (4) verbunden ist, wobei es ein hydraulisches Verbindungssystem (80, 81) ermöglicht, die Leitungen des Kreislaufs (der Kreisläufe) des bordeigenen Behandlungssystems mit dem beweglichen Teil (5) mit den Leitungen des Kreislaufs (der Kreisläufe) des Behandlungstanks in der Gebrauchsposition (P1) der Behandlungsmaschine zu verbinden und sie zu trennen, wenn der bewegliche Teil (5) entfernt wird.

4. Maschine nach einem der Ansprüche 1 bis 3, bei der die Trennwandschnittstelle (4) eine Trennwand (41) umfasst, die dazu bestimmt ist, sich entlang der Öffnung der Wand zu erstrecken, indem sie diese verschließt, sowie ein peripheres Befestigungssystem (42), das fest mit der Trennwand verbunden ist und sich entlang des Randes der Öffnung der Wand, entlang ihrer Ständer oder sogar entlang ihres oberen Randes erstreckt.

5. Maschine nach einem der Ansprüche 1 bis 4, wobei das periphere Befestigungssystem (42) Folgendes umfasst:
- ein erstes Element (43), bezeichnet als Stützelement, das dazu bestimmt ist, sich auf einer Seite der Wand abzustützen,
- ein zweites Element (44), bezeichnet als Gegenstützelement, das dazu bestimmt ist, sich auf der anderen Seite der Wand abzustützen,
- ein Einstell- und/oder Klemmsystem (45), zum Beispiel ein Schraubensystem, das so konfiguriert ist, dass es den Abstand zwischen dem ersten Element (43) und dem zweiten Element (44) auf die Dicke der Wand (M) einstellt oder sogar ermöglicht, dass die Wand zwischen dem ersten Element und dem zweiten Element eingeklemmt werden kann.

6. Maschine nach Anspruch 4 oder 5, wobei die Trennwandschnittstelle (4) einen Umfangsrahmen (9) mit einer Maskierungsfunktion aufweist, der dazu bestimmt ist, sich von der Fläche der Trennwandschnittstelle aus zu erstrecken, gegen die der Rahmen des beweglichen Teils (5) in der Gebrauchsposition P1 zu positionieren ist;
wobei die Innenwände des Rahmens (9) entlang der Ränder der Öffnung der Wand (M), indem sie diese abdecken oder sogar über die Fläche der Wand auf der Seite des beweglichen Teils (5) hinausragen, eine Aufnahme für den abnehmbaren Teil bilden, deren Breitenmaß an das Maß des beweglichen Teils (5) angepasst ist.

7. Maschine nach einem der Ansprüche 1 bis 6, wobei der selbsttragende Rahmen des beweglichen Teils (5) eine Einrichtung ist, die dazu bestimmt ist, in der genannten Gebrauchsposition (P1) auf dem Boden (S) zu stehen.

8. Maschine nach Anspruch 7, wobei der selbsttragende Rahmen des beweglichen Teils (5) an seinem unteren, vorzugsweise feststellbaren Teil Rollenelemente (10), wie zum Beispiel Rollen, und auch omnidirektionale Räder aufweist, mit denen der bewegliche Teil (5) durch ein Rollen am Boden (S) von der Maschine entfernt werden kann.

9. Anordnung, umfassend eine Behandlungsmaschine (1) nach einem der Ansprüche 1 bis 8 und eine Wand (M), die ein erstes Gehäuse (Sg) mit einer geregelten/nicht geregelten Atmosphäre von einem zweiten Gehäuse (Sb) mit einer geregelten/nicht geregelten Atmosphäre trennt, das eine Durchgangsöffnung (O_{M}), aufweist, und wobei die Trennwandschnittstelle dauerhaft an der Wand befestigt ist, die so konfiguriert ist, dass sie die Öffnung der Wand verschließt, und zwar derart, dass während der Wartung des Behandlungssystems das Entfernen des beweglichen Teils (5) der Behandlungsmaschine möglich ist, während die Trennschnittstelle (4) der Behandlungsmaschine noch die Öffnung (O_{M}) der Wand verschließt, wobei die Öffnung (40) der Trennschnittstelle dann durch das zweite Türsystem (31) verschlossen wird.

10. Verfahren zur Wartung einer Behandlungsmaschine (1) einer Anordnung nach Anspruch 9, wobei die Wartung des Behandlungssystems durch Ausführung der folgenden Schritte durchgeführt wird:
- Entfernen des beweglichen Teils (5) der Behandlungsmaschine, während die genannte Trennwandschnittstelle (4) der Behandlungsmaschine weiterhin die Öffnung (Om) der Wand verschließt, wobei die Öffnung (40) der Trennwandschnittstelle dann durch das zweite Türsystem (31) verschlossen wird,
- Transportieren des beweglichen Teils zu einer Werkstatt für Fernwartung und Überholung/Reparatur des genannten Behandlungssystems,
- Zurückbringen des überholten/reparierten beweglichen Teils (5) und Anordnen in seiner Gebrauchsposition (P1) in unmittelbarer Nähe der Trennwandschnittstelle (4).

11. Anordnung, umfassend eine Maschine (1') zur Behandlung eines medizinischen Geräts, wie zum Beispiel eines Endoskop-Reinigungs- und Desinfektionsgeräts, und eine Trennwand, wobei die Maschine an einer Wand, bezeichnet als Trennwand, auf der Höhe einer Durchgangsöffnung der Trennwand integriert ist, indem sie diese verschließt, wobei die Wand ein erstes Gehäuse (Sg) mit einer geregelten/nicht geregelten Atmosphäre von einem zweiten Gehäuse (Sb) mit einer geregelten/nicht geregelten Atmosphäre trennt, wobei die Maschine auf demselben selbsttragenden Rahmen Folgendes umfasst:
- einen Behandlungstank (2') mit einer ersten Öffnung, die so konfiguriert ist, dass sie das Be-/Entladen eines medizinischen Geräts aus dem ersten Gehäuse ermöglicht, und einer zweiten Öffnung, die so konfiguriert ist, dass sie das Be-/Entladen eines medizinischen Geräts aus dem zweiten Gehäuse ermöglicht,
- ein erstes Türsystem, das die Abdichtung der ersten Öffnung sicherstellt,
- ein zweites Türsystem (31'), das die Abdichtung der zweiten Öffnung sicherstellt,
- ein Behandlungssystem für ein medizinisches Gerät mit einem oder mehreren Behandlungskreisläufen, einschließlich einer oder mehrerer Pumpen, einem oder mehreren Magnetventilen, zur Behandlung der Innen- und Außenflächen des in dem Behandlungstank enthaltenen medizinischen Geräts durch Eintauchen und/oder Besprühen mittels einer Desinfektionslösung, die aus einem Tank außerhalb des Behandlungstanks entnommen wird,
- ein programmierbares Automatensystem zur Steuerung des Behandlungssystems, insbesondere des Motors und der Magnetventile des Systems, das für die Durchführung eines Desinfektionszyklus eines medizinischen Geräts konfiguriert ist, **dadurch gekennzeichnet, dass** die Anordnung ein Türsystem (100) umfasst, gelenkig an der Wand befestigt ist, die so konfiguriert ist, dass sie sich von einer ersten Öffnungsposition (P3), in der die Tür die Öffnung der Wand (M) freigibt, in die Gebrauchsposition der Maschine, in der der selbsttragende Rahmen der Maschine gegen die Öffnung der Wand positioniert ist, indem er sie schließt, in eine zweite Schließposition (P4) bewegt, in der die Öffnung der Wand durch das Wandsystem nach dem Entfernen der Behandlungsmaschine geschlossen werden kann.

## Claims

1. A treatment machine (1) for a medical device, such as an endoscope washer-disinfector, that is intended to be integrated into a wall (M), referred to as a separation wall, at a through opening (O_{M}) in the separation wall, the wall being intended to separate a first enclosure (Sg) having a controlled/uncontrolled atmosphere from a second enclosure (Sb) having an uncontrolled/controlled atmosphere, said machine comprising:
- a treatment tank (2) having a first opening (20) that is configured to allow loading/unloading of a medical device from the first enclosure (Sg) and a second opening (21) that is configured to allow loading/unloading of a medical device from the second enclosure (Sb),
- a first door system (30) that tightly seals the first opening (20),
- a second door system (31) that tightly seals the second opening (21),
- a treatment system for a medical device having one or more treatment circuits, including one or more pumps and one or more solenoid valves, allowing treatment of the interior and exterior surfaces of the medical device contained in the treatment tank (2) through immersion in and/or spraying of disinfectant solution taken from a reservoir outside the treatment tank,
- a programmable logic controller system that controls the treatment system, particularly the motor and the solenoid valves of the system, and is configured for the implementation of a disinfection cycle of a medical device,
**characterized in that** said machine (1) comprises:
- a partitioning interface (4) that is intended to be permanently fixed to the wall (M) and configured so as to close the opening (O_{M}) in the wall, said partitioning interface comprising at least one through opening (40) that is intended to be opposite the second opening in the tank, at least when the treatment machine is in the operating position (P1), the second door system (31) being secured to said partitioning interface (4), ensuring, in its closed position, the closure of said opening (40) in the partitioning interface,
- a movable part (5) that can be removed from the partitioning interface (4) and comprises a self-supporting chassis and at least partially includes the pump(s) and solenoid valves of said treatment system of the medical device as well as said programmable logic controller, which, when the treatment machine is in the operating position (P1), is configured to position itself in the immediate vicinity of the partitioning interface (4), namely in such a way as to allow, during maintenance on the treatment system, the removal of the mobile part (5) of the treatment machine while said partitioning interface (4) of the treatment machine continues to close the opening in the wall, the opening (40) in the partitioning interface (4) then being closed by the second door system (31).

2. The machine according to claim 1, wherein the mobile part (5) includes the treatment tank (2) and a joint system (7) that is secured to the mobile part (5) and/or secured to the partitioning interface (4) and ensures a seal between the second opening (21) in the tank and the corresponding opening in the partitioning interface when the treatment machine is in the operating position (P1), for which purpose the second opening (21) is pressed against the partitioning interface by means of said joint system, enabling the removal of the mobile part when removed.

3. The machine according to claim 1, wherein the treatment tank (2) is secured to the partitioning interface (4), a hydraulic connection system (80, 81) making it possible to connect the pipes of the circuit or circuits of the treatment system included by the mobile part (5) to the pipes of the circuit(s) of the treatment tank when the treatment machine is in said use position (P1) and to disconnect them during removal of the mobile part (5).

4. The machine according to one of claims 1 to 3, wherein the partitioning interface (4) comprises a partition (41) that is intended to extend along the opening in the wall while closing said opening, as well as a peripheral fixing system (42) that is secured to the partition and extends along the edge of the opening in the wall, along its uprights, or its upper edge.

5. The machine according to one of claims 1 to 4, wherein the peripheral fixing system (42) comprises:
- a first element (43), referred to as a support, that is intended to abut one side of the wall,
- a second element (44), referred to as a counter-support, that is intended to abut the other side of the wall,
- an adjustment and/or tightening system (45), for example in the form of a screw, that is configured so as to adjust the spacing between the first element (43) and the second element (44) to the thickness of the wall (M) or to allow the wall to be clamped between the first element and the second element.

6. The machine according to either claim 4 or claim 5, wherein the partitioning interface (4) comprises a peripheral frame (9) having the function of a cover that is intended to extend from the face of the partitioning interface against which the chassis of the movable part (5) is intended to be positioned in the use position P1;
along the edges of the opening in the wall (M) while covering them, or projecting beyond the face of the wall on the mobile-part side (5), the internal walls of the frame (9) forming a housing for the removable part, the width dimension of which housing is adjusted to the dimension of the movable part (5).

7. The machine according to one of claims 1 to 6, wherein the self-supporting chassis of the movable part (5) is a piece of furniture that is intended to be supported on the ground (S) in said use position (P1).

8. The machine according to claim 7, wherein the self-supporting chassis of the movable part (5) comprises rolling members (10), such as rollers or omnidirectional wheels, at its lower part that can preferably be locked, allowing the moving part (5) of the machine to be removed by rolling it on the ground (S).

9. An assembly comprising a treatment machine (1) according to one of claims 1 to 8, and a wall (M) separating a first enclosure (Sg) having a controlled/uncontrolled atmosphere from a second enclosure (Sb) having an uncontrolled/controlled atmosphere, having a through opening (O_{M}), and in which said partitioning interface is permanently fixed to the wall and configured so as to close the opening in the wall and in such a way as to allow, during maintenance on the treatment system, the removal of the mobile part (5) of the treatment machine while said partitioning interface (4) of the treatment machine continues to close the opening (O_{M}) in the wall, the opening (40) in the partitioning interface then being closed by the second door system (31).

10. A method of maintaining a treatment machine (1) of an assembly according to claim 9, wherein the maintenance of the treatment system is carried out by implementing the following steps:
- removing the mobile part (5) of the treatment machine while said partitioning interface (4) of the treatment machine continues to close the opening (Oₘ) in the wall, said opening (40) in the partitioning interface then being closed by the second door system (31),
- transporting the mobile part to a remote maintenance workshop and inspecting/repairing said treatment system,
- returning the inspected/repaired moving part (5) and placing said moving part in its use position (P1) in the immediate vicinity of the partitioning interface (4).

11. An assembly comprising a treatment machine (1') of a medical device, such as an endoscope washer-disinfector, and a separation wall, said machine being integrated into a wall, referred to as a separation wall, at a through opening in the separation wall while closing said through opening, the wall separating a first enclosure (Sg) having a controlled/uncontrolled atmosphere from a second enclosure having an uncontrolled/controlled atmosphere (Sb), said machine comprising, on the same self-supporting chassis:
- a treatment tank (2') having a first opening that is configured to allow loading/unloading of a medical device from the first enclosure and a second opening that is configured to allow loading/unloading of a medical device from the second enclosure,
- a first door system that tightly seals the first opening,
- a second door system (31') that tightly seals the second opening,
- a treatment system for a medical device having one or more treatment circuits, including one or more pumps and one or more solenoid valves, allowing the treatment of the interior and exterior surfaces of the medical device contained in the treatment tank through immersion in and/or spraying of disinfectant solution taken from a reservoir outside the treatment tank,
- a programmable logic controller system that controls the treatment system, particularly the motor and the solenoid valves of the system, and is configured for the implementation of a disinfection cycle of a medical device, **characterized in that** said assembly comprises a door system (100) that is hinged to the wall and is configured to pass from a first open position (P3), for which said door releases the opening in the wall (M), into the use position of the machine, for which the self-supporting chassis of the machine is positioned against the wall opening, closing it toward a second closed position (P4), allowing the wall opening to be closed by the wall system after removal of the treatment machine.
